# EUROPÄISCHE PATENTSCHRIFT

(11) **EP 1 042 259 B1**
(45) Veröffentlichungstag und Bekanntmachung des Hinweises auf die Patenterteilung: **13.03.2002**
(21) Anmeldenummer: 98963560.2
(22) Anmeldetag: 10.12.1998
(51) Int. Cl.: C07C 29/149

(54) **VERFAHREN ZUR HERSTELLUNG VON 1,6-HEXANDIOL**
METHOD FOR PRODUCING 1,6-HEXANEDIOL
PROCEDE POUR LA PREPARATION DE 1,6-HEXANEDIOL

(30) Priorität: 23.12.1997 DE 19757554
(43) Veröffentlichungstag der Anmeldung: 11.10.2000
(73) Patentinhaber: BASF AKTIENGESELLSCHAFT, 67056 Ludwigshafen (DE)
(72) Erfinder: BREITSCHEIDEL, Boris, D-67117 Limburgerhof (DE); PINKOS, Rolf, D-67098 Bad Dürkheim (DE); STEIN, Frank, D-67098 Bad Dürkheim (DE); LIANG, Shelue, D-67071 Ludwigshafen (DE); FISCHER, Rolf, Hartmuth, D-69121 Heidelberg (DE)
(86) Internationale Anmeldenummer: EP9808072
(87) Internationale Veröffentlichungsnummer: WO9933773

(56) Entgegenhaltungen:
- DE-A- 2 609 145
- DE-A- 4 141 199

## Beschreibung

Die Erfindung betrifft ein verbessertes Verfahren zur Herstellung von 1,6-Hexandiol durch Gasphasen-Hydrierung von Adipinsäurediestern, 6-Hydroxycapronsäureestern oder deren Gemischen in Gegenwart von chromfreien, im wesentlichen Kupfer, Mangan und Aluminium enthaltenden Katalysatoren oder von Raney-Kupfer unter Einhaltung bestimmter Hydrierbedingungen.

Aus WO 97/31882, Beispiel 1, ist bekannt, Gemische aus Adipinsäuredimethylester und 6-Hydroxycapronsäuremethylester in der Flüssigphase bei 220°C/220 bar in Gegenwart von 70 Gew.-% CuO, 25 Gew.-% ZnO und 5 Gew.-% Al₂O₃ enthaltenden Katalysatoren mit Selektivitäten von über 99 % (Umsatz 99,5 %) zu Hexandiol zu hydrieren. Nachteilig an dieser Hydrierung in der Flüssigphase ist der hohe Reaktionsdruck, der beträchtliche Investitionskosten für die Hydrieranlagen nach sich zieht. Dieser Nachteil kann durch Hydrieren in der Gasphase ausgeglichen werden, da bei Esterhydrierungen im allgemeinen deutlich niedrigere Reaktionsdrucke, z.B. Drucke unterhalb von 100 bar, ausreichen. Eine Bedingung für die Wirtschaftlichkeit derartiger Gasphasenhydrierungen ist jedoch, daß der Investitionskostenvorteil nicht durch andere Kostenfaktoren wieder aufgezehrt wird. Daher muß bei der Gasphasenhydrierung eine ähnlich hohe Hexandiol-Selektivität wie bei der Flüssigphasen-Hydrierung erreicht werden.

Hieraus ergab sich die Aufgabe, ein Verfahren zur Gasphasenhydrierung von Adipinsäurediestern, 6-Hydroxycapronsäureestern oder Gemischen aus Adipinsäurediestern und 6-Hydroxycapronsäureestern in Gegenwart von überwiegend Kupfer enthaltenden Katalysatoren zu Hexandiol zu finden, das bei C₆-Ester-Umsätzen von mindestens 90 %, insbesondere mindestens 95 %, Hexandiol-Selektivitäten von mindestens 95 %, insbesondere mehr als 98 % ermöglicht.

Bekannt war aus der japanischen Offenlegungsschrift S 64-85938, Adipinsäuredimethylester oder Adipinsäurediethylester bei 160 - 250°C und 10 bis 70 Atmosphären und Diester/Wasserstoff-Molverhältnissen von 1 : 100 bis 1 : 590 in der Gasphase in Gegenwart von Kupferchromit-Katalysatoren zu Hexandiol zu hydrieren. Nur in einem von elf Patentbeispielen wird eine Hexandiol-Selektivität von über 98 % erreicht: In Beispiel 6 wird eine Hexandiol-Selektivität von 98,9 % (Umsatz 97,5 %) erzielt. Allerdings führt dabei das Wasserstoff/Diester-Molverhältnis von 457 zu sehr hohen Energiekosten. Schließlich sind chromhaltige Katalysatoren wegen der Toxizität von Chrom problematisch. Desaktivierte Katalysatoren müssen unter hohem Kostenaufwand sicher deponiert werden.

Aus U.S. 5.395.990, Beispiel 13, geht hervor, daß man bei der Gasphasen-Hydrierung von Adipinsäuredimethylester bei 180°C und 62 bar, einem Wasserstoff/Diester-Molverhältnis von 480 und einer Katalysator-Belastung von 0,4 1 Diester/l_{Katalysator • Stunde} in Gegenwart eines aus Kupfer (41,1 Gew.-%), Mangan (6,2 Gew.-%) und Aluminium (20,4 %) bestehenden Katalysators ähnliche Ergebnisse wie in Beispiel 11 erhält. Dort wird jedoch für die Gasphasen-Hydrierung von Maleinsäuredimethylester, die unter gleichen Bedingungen wie in Beispiel 13 durchgeführt wurde, keine Angabe zur Butandiol-Selektivität gemacht.

Aus U.S. 5.406.004, Beispiel 13, ist bekannt, Adipinsäuredimethylester bei 220°C und 62 bar, einem Diester/Wasserstoff-Molverhältnis zwischen 248 und 383 und einer Katalysator-Belastung von 0,4 1 Diester/l_{Katalysator • Stunde} in Gegenwart des in U.S. 5.395.990, Beispiel 13 genannten Katalysators zu Hexandiol zu hydrieren. Eine Angabe zur Hexandiol-Selektivität und zum Diester-Umsatz wird nicht gemacht. Es wird lediglich festgestellt, daß ähnliche Ergebnisse wie in den Beispielen 2 bis 4 erzielt werden. Allerdings handelt es sich bei diesen Beispielen nicht um eine reine Gasphasen-Hydrierung, da die Temperatur des den Reaktor verlassenden Reaktionsgemisches unterhalb des Taupunktes der Mischung liegt.

Bei Nacharbeitung der Beispiele 13 in U.S. 5.395.990 und U.S. 5 406 004 wurde festgestellt, daß die Hexandiol-Selektivitäten deutlich unter den hier geforderten 95 % bzw. 98 % liegen (Vergleichsbeispiele 14 und 15). Dabei sind es vor allem zwei Gruppen von Nebenprodukten, die für die mangelnde Hexandiol-Selektivität verantwortlich sind:
a) 5-Ringverbindungen:
   2-Methylcyclopentanol (1), 2-Methylcyclopentanon (2), Cyclopentanol (3) und Hydroxymethylcyclopentan (4), die ausgehend von Adipinsäuredimethylester entstehen können: 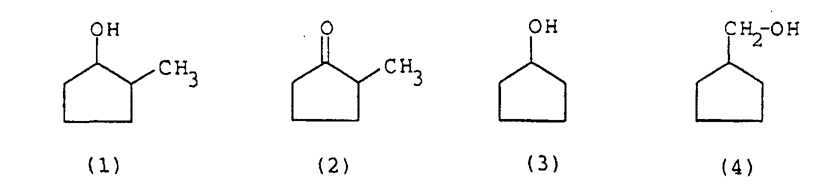
   Das bei der Gasphasenhydrierung von Adipinsäuredimethylester freigesetzte Methanol könnte als Methylierungsmittel fungieren.
   Bezogen auf die Summe der 5-Ringverbindungen werden z.B. in Beispiel 5 61 % (1), 29 % (2), 6 % (3) und 4 % (4) gefunden.
   Gemäß dem Verfahren der EP-A 251 111, wird sogar die Bildung von Cyclopentanon zum Hauptprodukt, wenn man Adipinsäurediester bei 300 bis 345°C in der Gasphase an festen oxidischen Katalysatoren von Elementen der I. bis V. Hauptgruppe, der I. bis VIII. Nebengruppe des Periodischen Systems der Elemente oder Oxiden der seltenen Erdmetalle, insbesondere Aluminiumoxid, umsetzt.
b) C₁₂- und C₁₃-Ester
   Durch Umesterung von 6-Hydroxycapronsäuremethylester mit Hexandiol entsteht 6-Hydroxy-capronsäure(6-hydroxyhexyl)ester (5). 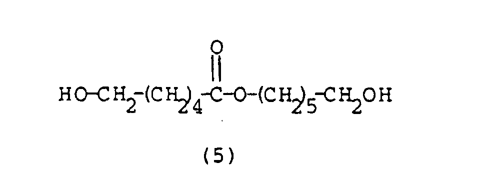
   In deutlich geringerer Menge als (5) wird Adipinsäuremethyl(6-hydroxyhexyl)ester (6) beobachtet, der wohl durch Umesterung von Adipinsäuredimethylester mit Hexandiol entsteht. 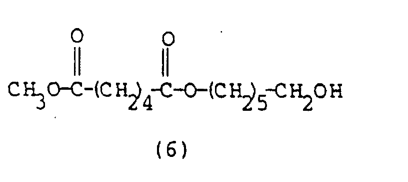
   Das Molverhältnis von (5) zu (6) liegt etwa bei 90 : 10.

Das mengenmäßig dominierende Nebenprodukt (5) besitzt ein wesentlich höheres Molekulargewicht (MG 232) und damit einen deutlich höheren Siedepunkt als Adipinsäuredimethylester (MG 174) und 6-Hydroxycapronsäuremethylester (MG 146).

Je mehr (5) und (6) gebildet werden, desto höhere Temperaturen und/oder Wasserstoffmengen sind notwendig, um (5) und (6) in die Gasphase zu bringen und dort zu hydrieren. (5) und (6) nach der Hydrierung aus dem Hydrieraustrag abzutrennen und zurückzuführen, ist aufwendig. Daher müssen beide, sofern sie nicht hydriert werden, als Nebenprodukte angesehen werden.

Die schon beschriebene Aufgabe wird nun erfindungsgemäß gelöst mit einem Verfahren zur Herstellung von 1,6-Hexandiol durch Hydrierung von Adipinsäurediestern und/oder 6-Hydroxycapronsäureestern in der Gasphase bei erhöhter Temperatur und erhöhtem Druck in Gegenwart von chromfreien Katalysatoren, wobei man die Hydrierung
a) unter Verwendung von als wesentliche Bestandteile Kupfer, Mangan und Aluminium enthaltenden Katalysatoren oder in Gegenwart von Raney-Kupfer,
b) bei Temperaturen von 150 bis 230°C und Drücken von 10 bis 70 bar,
c) bei einem Molverhältnis Wasserstoff zu hydrierendem Ester von 150 zu 1 bis 300 zu 1 und
d) bei einer Katalysatorbelastung von 0,01 bis 0,3 kg C₆-Ester pro Liter Katalysator und Stunde durchführt.

Es war überraschend, daß es gelingt, die Summe der Nebenprodukte aus 5-Ringverbindungen und C₆-Säure(6-hydroxyhexyl)estern unter 5 Mol-%, insbesondere 2 Mol-% (bezogen auf eingesetzten Adipinsäuredi- und 6-Hydroxycapronsäureester) zu halten und damit eine Hexandiol-Selektivität von mindestens 95 %, insbesondere mindestens 98 % zu erreichen:

Bei der Gasphasen-Hydrierung von Adipinsäuredimethyl- und 6-Hydroxycapronsäuremethylester entstehen, wie schon ausgeführt, wahrscheinlich als Folgeprodukte von Cyclopentanon-2-carbonsäuremethylester und Cyclopentanon die Nebenprodukte 2-Methylcyclopentanol, 2-Methylcyclopentanon, Cyclopentanol und Hydroxymethylcyclopentan. Diese Nebenprodukte sind charakteristisch für die Hydrierung von C6-Mono- und Dicarbonsäureestern. Sie werden daher bei der Hydrierung von alpha, omega-C₄-, C₅-, C₇- und C₈-Diestern nicht beobachtet. Ihre Menge nimmt mit steigender Temperatur zu.

Außerdem entstehen bei der Gasphasen-Hydrierung von Adipinsäurediestern hochsiedende 6-Hydroxycapronsäure(6-hydroxyhexyl)ester und Adipinsäuremethyl(6-hydroxyhexyl)ester. Sie werden mit steigender Temperatur zunehmend unter Bildung des Wertproduktes Hexandiol hydriert.

Überraschenderweise wurde gefunden, daß die gewünschte Selektivität von 95 % bzw. 98 % trotz der Temperaturgegenläufigkeit der beiden Nebenproduktgruppen erreichbar ist.

Es war weiterhin nicht vorauszusehen, daß der Katalysator trotz der Nebenproduktbildung eine hohe Standzeit erreichen würde.

Ferner wurde bei Verwendung von nach DE-A 19 607 954 hergestellten Adipinsäurediester/6-Hydroxycapronsäureestergemischen, die noch zahlreiche andere Verbindungen enthalten, überraschenderweise gefunden, daß man sowohl hohe Hexandiol-Selektivitäten als auch lange Katalysator-Standzeiten erzielt.

Als Ausgangsstoffe für das erfindungsgemäße Verfahren können reine Adipinsäureester, z.B. C₁-C₄-Dialkylester, 6-Hydroxycapronsäureester, z.B. C₁-C₄-Alkylester oder Gemische davon eingesetzt werden. Bevorzugt kann man Estergemische einsetzen, wie sie bei der Veresterung von bei der Oxidation von Cyclohexan zu Cyclohexanon/Cyclohexanol als Nebenprodukte entstehenden Carbonsäuregemischen mit C₁-C₄-Alkoholen nach DE-A 19 607 954 gewonnen werden können. Diese Gemische können z. B. zusätzlich Glutarsäurediester, 5-Hydroxyvaleriansäureester, 2-Oxo-capronsäureester und Dihydromuconsäurediester enthalten.

Für das erfindungsgemäße Verfahren werden bevorzugt Methyl- und Ethylester der oben genannten Carbonsäuren als Ausgangsstoffe verwendet.

Die Hydrierung erfolgt katalytisch in der Gasphase.

Als Katalysatoren kommen chromfreie im wesentlichen Kupfer, Mangan und Aluminium enthaltende Katalysatoren in Betracht, die zusätzlich untergeordnete Mengen an Zink, Zirkon und/oder Silicium enthalten können.

Hierzu gehören vor allem Katalysatoren, wie sie in EP 0 552 463 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c ≧ 0, d > 0, a > b/2, b > a/4, a > c, a > d gilt und x die zur Wahrung der Elektroneutralität pro Formeleinheit erforderliche Anzahl von Sauerstoffionen bezeichnet. Im einzelnen kommen z.B. Katalysatoren der Zusammensetzungen von ungefähr 70 Gew.-% CuO, 20 Gew.-% Al₂O₃ und 10 Gew.-% Mn₂O₃ in Betracht.

Die Herstellung dieser Katalysatoren kann beispielsweise nach den Angaben der EP 0 552 463 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so daß man als besonders bevorzugte Fällungsmittel Alkalicarbonate oder Ammoniumcarbonate einsetzt. Wichtig für die Herstellung der Katalysatoren ist die thermische Behandlung der Zwischenstufen bei Temperaturen zwischen 500 °C und 1000 °C. Die BET-Oberfläche der Katalysatoren liegt zwischen 10 und 150 m²/g.

Alternativ kommt als Katalysator Raney-Kupfer in Betracht, das in an sich bekannter Weise durch Behandeln von Kupfer-Aluminium-Legierungen mit Alkalihydroxiden hergestellt werden kann und in stückiger Form verwendet wird.

Die Katalysatoren können in einem Festbett- oder einem Wirbelbettreaktor angeordnet sein. Die Hydrierung kann in Rieselfahrweise oder in Sumpffahrweise, durchgeführt werden. Dabei wird zweckmäßig mindestens so viel Wasserstoff als Hydriermittel und Trägergas verwendet, daß Edukte, Zwischenprodukte und Produkte während der Reaktion nie flüssig werden. Der überschüssige Wasserstoff wird vorzugsweise im Kreis geführt, wobei ein kleiner Teil als Abgas zur Entfernung von Inerten wie z. B. Methan ausgeschleust werden kann. Es können dabei ein Reaktor oder mehrere Reaktoren hintereinander oder parallel zueinander geschaltet verwendet werden.

Die Hydriertemperatur beträgt 150°C bis 230°C, bevorzugt 160°C bis 200°C, besonders bevorzugt 170°C bis 190°C.

Der Reaktionsdruck beträgt 10 bar bis 70 bar, bevorzugt 20 bar bis 60 bar, besonders bevorzugt 30 bar bis 50 bar.

Das Molverhältnis von Wasserstoff zur Summe der eingesetzten C₆-Ester beträgt 150 bis 300 zu eins, bevorzugt 170 bis 290 zu eins, besonders bevorzugt 180 bis 280 zu eins.

Die Katalysator-Belastung beträgt 0,01 bis 0,3, bevorzugt 0,05 bis 0,2, besonders bevorzugt 0,05 bis 0,15 kg zu hydrierende C₆-Ester/l_{Katalysator • Stunde}.

Der Umsatz, bezogen auf die Summe der Hexandiol bildenden C₆-Verbindungen wie Adipinsäurediester, 6-Hydroxycapronsäureester, Caprolacton und Dihydromuconsäurediester, soll dabei über 90 %, insbesondere über 95 % betragen.

Die Hydrierung wird zweckmässig kontinuierlich durchgeführt. Die Hydrierausträge werden nach der Kondensation des Hydrieraustrags bevorzugt destillativ aufgearbeitet.

Der Hydrieraustrag besteht im wesentlichen aus 1,6-Hexandiol und dem der Estergruppe entsprechenden Alkohol. Weitere Bestandteile sind vor allem, falls nach DE-A 19607954 hergestellte Estergemische eingesetzt werden, 1,5-Pentandiol, 1,4-Butandiol, 1,2-Cyclohexandiole sowie Monoalkohole mit 1 bis 6 C-Atomen und Wasser.

Bei unvollständigem Umsatz im Hydrieraustrag enthaltene Ausgangsverbindungen können destillativ abgetrennt und in die Hydrierstufe zurückgeführt werden.

### Beispiele

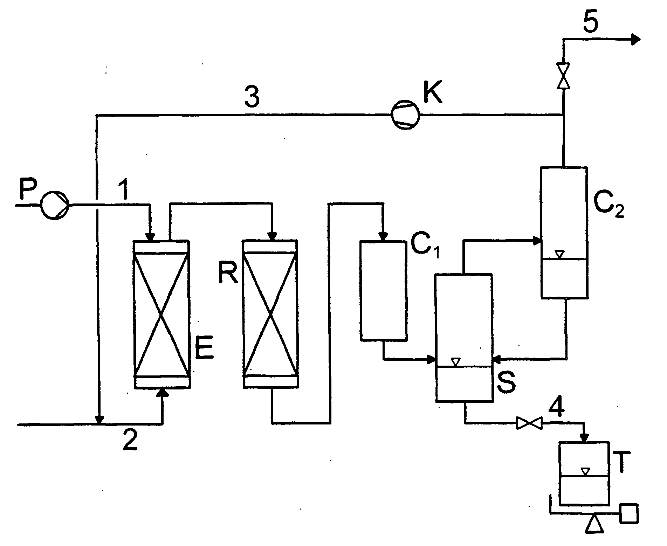

A) Hydrierapparatur:
   Die Versuche wurden in der in Fig. 1 schematisch dargestellten Hydrierapparatur kontinuierlich durchgeführt. Sie besteht aus einem Verdampfer (E), einem 1,4 1-Rohrreaktor (30 x 2000 mm) (R), zwei Kühlern C₁ und C₂ und einem Druckabscheider (S) zur Gewinnung der kondensierbaren Komponenten aus dem Wasserstoffstrom, einem Kreisgasverdichter (K) zur Rückführung des Kreisgaswasserstoffs (3) und einem Austragsgefäß (T) zum Sammeln des Reaktionsaustrags (4).

### Durchführung der Versuche:

In den Beispielen 1 - 12 und den Beispielen 14 und 15 wurde ein CuO (70 Gew.-%)/Mn203 (10 Gew.-%)/Al₂O₃ (20 Gew.-%)-Katalysator der Firma Südchemie (T4489) verwendet, der im Reaktor mit Wasserstoff/Stickstoffgemischen im Volumenverhältnis von 1 : 99 bis 100 : 0 bei 160 - 200 °C aktiviert wurde. Die Katalysatorzone wurde am oberen und unteren Ende durch eine Schicht Quarzringe begrenzt. In allen Fällen wurde mit Kreisgas gearbeitet, wobei 10 % des Kreisgases (5) ausgeschleust und durch die gleiche Menge Frischwasserstoff ersetzt wurden.

In Beispiel 13 wurde der Raney-Kupfer-Katalysator A 3900 der Firma Activated Metals verwendet.

Die C₆-Ester-Ausgangsverbindungen (1) wurden mit einer Pumpe (P) in den Verdampfer dosiert, wo sie verdampft und mit vorgeheiztem Wasserstoff (2) vermischt gasförmig in den Reaktor geleitet wurden. Das Molverhältnis Ester/Wasserstoff wurde durch Wägung der zugeführten Menge an Ausgangsprodukt und Messung der Wasserstoffströme ermittelt.

Der Hydrieraustrag wurde nach dem Kondensieren gewogen. Seine Zusammensetzung wurde gaschromatographisch unter Verwendung eines inneren Standards (Diethylenglykoldimethylether) quantitativ bestimmt. Jede Versuchseinstellung wurde etwa vier Tage ohne Änderung betrieben, erst dann wurde der Hydrieraustrag analysiert.

### Beispiele 1 - 12

Bei diesen Beispielen wurden 500 ml CuO/Mn₂O₃/Al₂O₃-Katalysator verwendet. Gearbeitet wurde mit reinem Adipinsäuredimethylester (Dichte nach Katalog der Firma Aldrich-Chemie, 1994, Steinheim, Seite 36: d = 1,063).

In Beispiel 12 wurde anstelle von Adipinsäuredimethylester ein nach DE-A 19 607 954 hergestelltes Gemisch aus Adipinsäuredimethylester, 6-Hydroxycapronsäuredimethylester und weiteren Estern verwendet. Es bestand aus 52,3 % Adipinsäuredimethylester, 9,1 % 6-Hydroxycapronsäuremethylester, 5.2 % Caprolacton, 4,1 % Dihydromuconsäuredimethylester, 1,5 % Bernsteinsäuredimethylester, 2,5 % Valerolacton, 2,2 % 5-Hydroxyvaleriansäureester, 2,7 % 2-Oxocapronsäureester und 6,4 % Glutarsäuredimethylester (alle Gew.-%).

### Beispiel 13 %

Gearbeitet wurde mit reinem Adipinsäuredimethylester. Als Katalysator wurden 500 ml Raney-Kupfer verwendet.

### Vergleichsbeispiele 14 - 16

In diesen Beispielen wurden Beispiel 13 von U.S. 5 395 990 (Vergleichsbeispiel 14) und Beispiel 13 von U.S. 5 406 004 (Vergleichsbeispiel 15: 220 °C; Molverhältnis Adipinsäuredimethylester/H₂ = 1 ; 248) nachgearbeitet. Verwendet wurden 150 ml CuO/Mn₂O₃/Al₂O₃-Katalysator.

Alle Versuchsergebnisse sind in der folgenden Tabelle 1 zusammengefaßt.

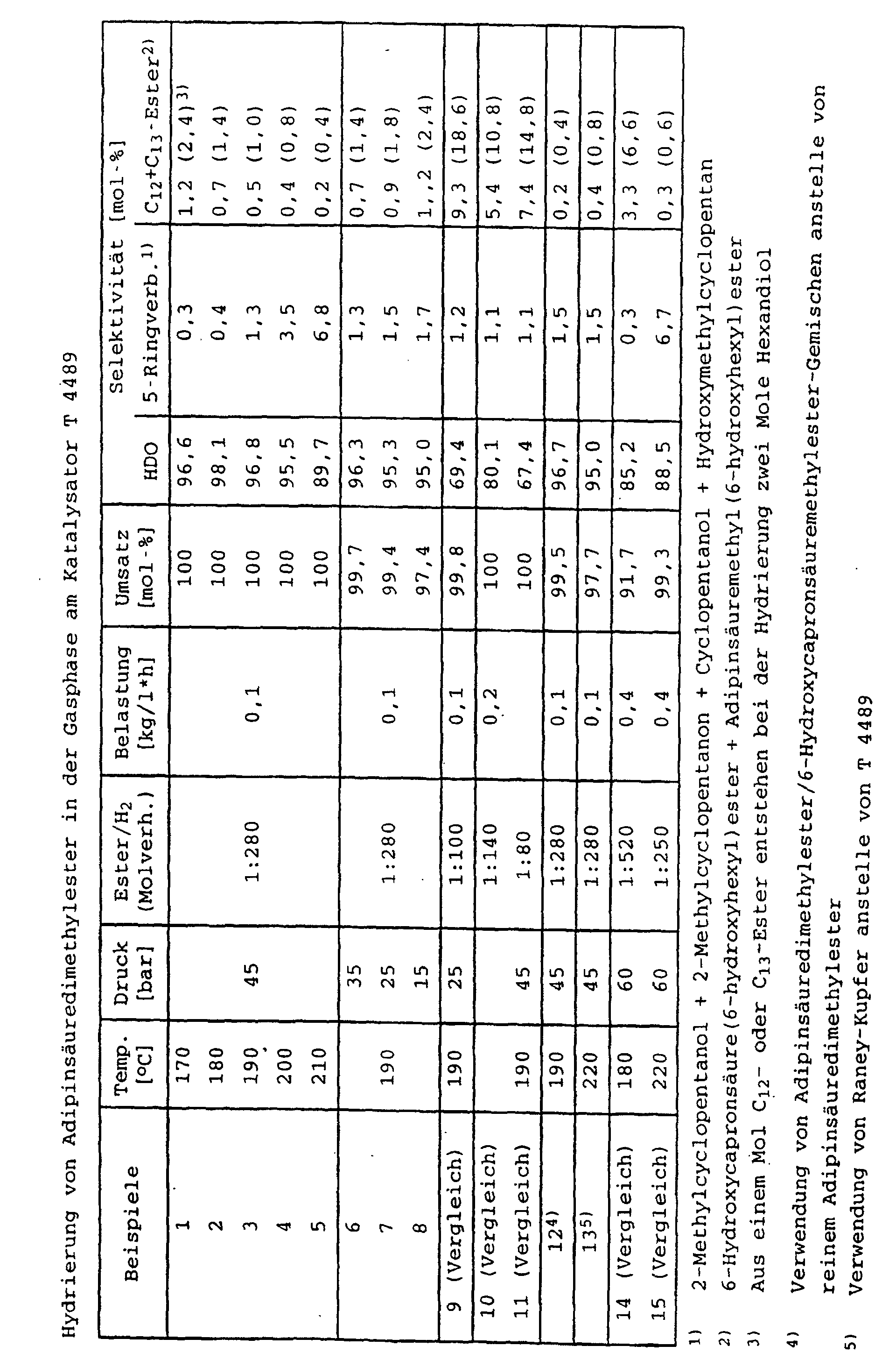

## Patentansprüche

1. Verfahren zur Herstellung von 1,6-Hexandiol durch Hydrierung von Adipinsäureestern und/oder 6-Hydroxycapronsäureestern in der Gasphase bei erhöhter Temperatur und erhöhtem Druck in Gegenwart von chromfreien Katalysatoren, **dadurch gekennzeichnet, daß** man die Hydrierung
a) unter Verwendung von als wesentliche Bestandteile Kupfer, Mangan und Aluminium enthaltenden Katalysatoren oder in Gegenwart von Raney-Kupfer,
b) bei Temperaturen von 150 bis 230°C und Drücken von 10 bis 70 bar,
c) bei einem Molverhältnis Wasserstoff zu hydrierendem Ester von 150 zu 1 bis 300 zu 1 und
d) bei einer Katalysatorbelastung von 0,01 bis 0,3 kg C₆-Ester pro Liter Katalysator und Stunde durchführt.

2. Verfahren gemäß Anspruch 1, **dadurch gekennzeichnet, daß** die Katalysatoren zusätzlich Zink, Zirkon und/oder Silicium enthalten.

3. Verfahren gemäß Anspruch 1, **dadurch gekennzeichnet, daß** man Katalysatorbelastungen von 0,05 bis 0,2 kg C₆-Ester/l_{Katalysator^{·}Stunde} einhält.

4. Verfahren gemäß Anspruch 1, **dadurch gekennzeichnet, daß** man Katalysatorbelastungen von 0,05 bis 0,15 kg C₆-Ester/l_{Katalysator^{·}Stunde} einhält.

5. Verfahren gemäß Anspruch 1, **dadurch gekennzeichnet, daß** man die Hydrierung mit Molverhältnissen von Wasserstoff zu C₆-Estern von 170 bis 290 zu eins durchführt.

6. Verfahren gemäß Anspruch 1, **dadurch gekennzeichnet, daß** man die Hydrierung mit Molverhältnissen von Wasserstoff zu C₆-Estern von 180 bis 280 zu eins durchführt.

7. Verfahren gemäß Anspruch 1, **dadurch gekennzeichnet, daß** man die Hydrierung bei Temperaturen von 160°C bis 200°C durchführt.

8. Verfahren gemäß Anspruch 1, **dadurch gekennzeichnet, daß** man die Hydrierung bei Temperaturen von 170 bis 190°C durchführt.

9. Verfahren gemäß Anspruch 1, **dadurch gekennzeichnet, daß** man die Hydrierung bei Drücken von 20 bis 60 bar durchführt.

10. Verfahren gemäß Anspruch 1, **dadurch gekennzeichnet, daß** man die Hydrierung bei Drücken von 30 bis 50 bar durchführt.

11. Verfahren gemäß Anspruch 1, **dadurch gekennzeichnet, daß** man Gemische aus Adipinsäurediestern und 6-Hydroxycapronsäureestern hydriert.

12. Verfahren gemäß Anspruch 1, **dadurch gekennzeichnet, daß** man als Ausgangsstoffe C₁-C₄-Mono- bzw. Dialkylester der 6-Hydroxycapronsäure bzw. Adipinsäure verwendet.

13. Verfahren gemäß Anspruch 1, **dadurch gekennzeichnet, daß** man als zu hydrierende Ausgangsstoffe Estergemische einsetzt wie sie durch Veresterung von Carbonsäuregemischen entstehen, die bei der Herstellung von Cyclohexanol/Cyclohexanon durch Oxidation von Cyclohexan mit Sauerstoff enthaltenden Gasen als Nebenprodukte erhalten werden.

## Claims

1. A process for producing 1,6-hexanediol by hydrogenation of adipic esters and/or 6-hydroxycaproic esters in the gas phase at elevated temperature and elevated pressure in the presence of chromium-free catalysts, which comprises hydrogenating
a) over a catalyst comprising copper, manganese and aluminum as essential constituents or over Raney copper,
b) at a temperature of from 150 to 230°C and a pressure of from 10 to 70 bar,
c) at a molar ratio of hydrogen to ester to be hydrogenated within the range from 150 : 1 to 300 : 1, and
d) at a catalyst space velocity of from 0.01 to 0.3 kg of C₆ ester per liter of catalyst per hour.

2. A process as claimed in claim 1, wherein the catalyst further comprises zinc, zirconium and/or silicon.

3. A process as claimed in claim 1, wherein the catalyst space velocity used ranges from 0.05 to 0.2 kg of C₆ ester per liter of catalyst per hour.

4. A process as claimed in claim 1, wherein the catalyst space velocity used ranges from 0.05 to 0.15 kg of C₆ ester per liter of catalyst per hour.

5. A process as claimed in claim 1, wherein the molar ratio of hydrogen to C₆ ester is within the range from 170 to 290:1.

6. A process as claimed in claim 1, wherein the molar ratio of hydrogen to C₆ ester is within the range from 180 to 280:1.

7. A process as claimed in claim 1, wherein the temperature is within the range from 160°C to 200°C.

8. A process as claimed in claim 1, wherein the temperature is within the range from 170 to 190°C.

9. A process as claimed in claim 1, wherein the pressure is within the range from 20 to 60 bar.

10. A process as claimed in claim 1, wherein the pressure is within the range from 30 to 50 bar.

11. A process as claimed in claim 1, wherein the ester to be hydrogenated comprises a mixture of adipic diester and 6-hydroxycaproic ester.

12. A process as claimed in claim 1, wherein the starting materials used are C₁-C₄-alkyl monoesters of 6-hydroxycaproic acid and/or C₁-C₄-alkyl diesters of adipic acid.

13. A process as claimed in claim 1, wherein the starting materials to be hydrogenated are ester mixtures as generated by esterification of carboxylic acid mixtures obtained as by-products in the production of cyclohexanol/cyclohexanone by oxidation of cyclohexane with oxygen-comprising gases.

## Revendications

1. Procédé de préparation de 1,6-hexanediol par hydrogénation d'esters de l'acide adipique et/ou d'esters de l'acide 6-hydroxycapronique en phase gazeuse à haute température et sous pression élevée, en présence de catalyseurs sans chrome, **caractérisé en ce qu'**on met en oeuvre l'hydrogénation
a) par utilisation de catalyseurs contenant, en tant que constituants essentiels, du cuivre, du manganèse et de l'aluminium, ou en présence de cuivre Raney,
b) à des températures de 150 à 230°C et sous des pressions de 10 à 70 bar,
c) en présence d'un rapport en moles de l'hydrogène à l'ester à hydrogéner de 150:1 à 300:1, et
d) en présence d'une charge de catalyseur de 0,01 à 0,3 kg d'ester d'acide en C₆ par litre de catalyseur et par heure.

2. Procédé selon la revendication 1, **caractérisé en ce que** les catalyseurs contiennent en outre du zinc, du zirconium et/ou du silicium.

3. Procédé selon la revendication 1, **caractérisé en ce qu'**on maintient des charges de catalyseur de 0,05 à 0,2 kg d'ester d'acide en C₆ par litre de catalyseur par heure.

4. Procédé selon la revendication 1, **caractérisé en ce qu'**on maintient des charges de catalyseur de 0,05 à 0,15 kg d'ester d'acide en C₆ par litre de catalyseur par heure.

5. Procédé selon la revendication 1, **caractérisé en ce qu'**on met en oeuvre l'hydrogénation avec des rapports en moles de l'hydrogène aux esters d'acides en C₆ de 170 à 290:1.

6. Procédé selon la revendication 1, **caractérisé en ce qu'**on met en oeuvre l'hydrogénation avec des rapports en moles de l'hydrogène aux esters d'acides en C₆ de 180 à 280:1.

7. Procédé selon la revendication 1, **caractérisé en ce qu'**on met en oeuvre l'hydrogénation à des températures de 160 à 200°C.

8. Procédé selon la revendication 1, **caractérisé en ce qu'**on met en oeuvre l'hydrogénation à des températures de 170 à 190°C.

9. Procédé selon la revendication 1, **caractérisé en ce qu'**on met en oeuvre l'hydrogénation sous des pressions de 20 à 60 bar.

10. Procédé selon la revendication 1, **caractérisé en ce qu'**on met en oeuvre l'hydrogénation sous des pressions de 30 à 50 bar.

11. Procédé selon la revendication 1, **caractérisé en ce qu'**on hydrogène des diesters de l'acide adipique et des esters de l'acide 6-hydroxycapronique.

12. Procédé selon la revendication 1, **caractérisé en ce qu'**on utilise en tant que substances de départ des esters mono- ou di-(alkyliques en C₁-C₄) de l'acide 6-hydroxycapronique ou de l'acide adipique.

13. Procédé selon la revendication 1, **caractérisé en ce que**, en tant que substances de départ à hydrogéner, on utilise des mélanges d'esters tels ceux qui sont obtenus par estérification de mélanges d'acides carboxyliques qui sont obtenus en sous-produits lors de la préparation de cyclohexanol/cyclohexanone par oxydation de cyclohexane avec des gaz oxygénés.
